(19) Europäisches Patentamt
European Patent Office
Office européen des brevets

(11) Veröffentlichungsnummer: **0 049 863**
**B1**

(12) # EUROPÄISCHE PATENTSCHRIFT

(45) Veröffentlichungstag der Patentschrift: **12.12.84**

(21) Anmeldenummer: **81108025.8**

(22) Anmeldetag: **07.10.81**

(51) Int. Cl.³: **C 07 C 27/06**, C 07 C 29/15, C 07 C 31/08, C 07 C 45/49, C 07 C 47/06, C 07 C 51/10, C 07 C 53/08 // B01J27/10

(54) **Verfahren zur Herstellung von Essigsäure, Acetaldehyd und Ethanol aus Synthesegas.**

(30) Priorität: **11.10.80 DE 3038448**

(43) Veröffentlichungstag der Anmeldung: **21.04.82 Patentblatt 82/16**

(45) Bekanntmachung des Hinweises auf die Patenterteilung: **12.12.84 Patentblatt 84/50**

(84) Benannte Vertragsstaaten: **BE DE FR GB IT NL**

(56) Entgegenhaltungen:
**DE - A - 2 814 427**
**DE - A - 2 850 201**

(73) Patentinhaber: **HOECHST AKTIENGESELLSCHAFT, Postfach 80 03 20, D-6230 Frankfurt am Main 80 (DE)**

(72) Erfinder: **Schmidt, Hans-Joachim, Dr., Am Burgenblick 6, D-6240 Königstein/Taunus (DE)**
Erfinder: **Leupold, Ernst Ingo, Dr., Am Zäunefeld 15, D-6392 Neu-Anspach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein Verfahren zur Herstellung von Essigsäure, Acetaldehyd und Ethanol durch Umsetzung von Kohlenmonoxid mit Waserstoff in der Gasphase an Rhodium und ggf. Promotoren enthaltenen Katalysatoren bei erhöhten Temperaturen und Drücken.

Derartige Verfahren sind bereits bekannt. Es ist auch bereits bekannt, daß der Zusatz von Promotoren die Aktivität erhöht und/oder die Selektivität zu den einzelnen Verbindungen beeinflußt. Verfahren dieser Art sind beschrieben in den deutschen Auslegeschriften 2 503 204, 2 503 233, 2 628 463, in den deutschen Offenlegungsschriften 2 628 576, 2 712 732, 2 814 365, 2 846 148 und in den US-Patentschriften 4 096 164, 4 101 450, 4 136 104, 4 162 262.

Bei diesen Verfahren ist die Katalysator-Lebensdauer jedoch häufig noch nicht befriedigend.

In den deutschen Offenlegungsschriften 2 825 495, 2 825 598, 2 850 110, 2 850 201 sind deshalb bereits Maßnahmen beschrieben worden, die zu einer verbesserten Lebensdauer der Katalysatoren führen und die darin bestehen, daß man Magnesiumsalze oder -verbindungen bzw. Halogenwasserstoffe oder -verbindungen kontinuierlich oder diskontinuierlich der Reaktionszone zuführt. Diese Maßnahmen haben eine wesentliche Stabilitätsverbesserung zur Folge, sind jedoch auf die genannten Promotoren beschränkt und erfordern im allgemeinen zusätzliche apparative Einrichtungen für die Dosierung und Verdampfung der Zusatzstoffe.

Es wurde nun gefunden, daß man die Lebensdauer der Rhodiumkatalysatoren in einfacher Weise und dadurch verbessern kann, daß man in der Anfangsphase des Verfahrens während des Aufheizens des Katalysators die Temperaturerhöhung in den letzten 75 bis 125° C vor Erreichen der Betriebstemperatur kontinuierlich oder in kleinen Stufen und in einem Zeitraum von 100 bis 1000 Stunden vornimmt. Duch diese Maßnahme wird nicht nur die Lebensdauer der Katalysatoren wesentlich verbessert, sondern es resultiert auch eine erhöhte Selektivität zu den gewünschten sauerstoffhaltigen $C_2$-Verbindungen, wobei insbesondere die Selektivität zu Essigsäure zunimmt und nach Erreichen der Betriebstemperatur konstant bleibt, während die Selektivität zu Methan anfangs etwas abfällt.

Gegenstand der Erfindung ist daher ein Verfahren zur Herstellung von Essigsäure, Acetaldehyd und Ethanol durch Umsetzung von Kohlenmonoxid und Wasserstoff in der Gasphase an Rhodium und ggf. Promotoren enthaltenden Katalysatoren bei erhöhten Temperaturen und erhöhten Drücken, das dadurch gekennzeichnet ist, daß man in der Anfangsphase des Verfahrens während des Aufheizens des Katalysators auf die für den Dauerbetrieb vorgesehene Betriebstemperatur $T_R$ die Temperaturerhöhung im Temperaturbereich zwischen $T_O$ und $T_R$, wobei $T_O$ 75 bis 125° C unterhalb $T_R$ liegt, kontinuierlich oder in Temperaturstufen von maximal 10° C und in einem Zeitraum von 100 bis 1000 Stunden vornimmt, wobei die Temperatur in einem beliebigen 10-Stunden-Intervall um maximal 10° C erhöht wird. Vorzugsweise wird die Temperaturerhöhung in einem Zeitraum von 120 bis 800 Stunden vorgenommen.

Es hat sich also gezeigt, daß in der Anfangsphase der Synthesegasumsetzung die Geschwindigkeit der Aufheizung für die Lebensdauer des Katalysators und die Selektivität zu den sauerstoffhaltigen $C_2$-Verbindungen von ausschlaggebender Bedeutung ist. Wesentlich ist, daß die Temperaturerhöhung in dem 75 bis 125° C unter der Betriebstemperatur $T_R$ liegenden Bereich in kleinen Stufen von maximal 10° C und in einem Zeitraum von 100 bis zu 1000 Stunden vorgenommen wird.

Das durch die erfindungsgemäße, verzögerte Aufheizungsgeschwindigkeit im Temperaturbereich $T_O$ bis $T_R$ eine Erhöhung sowohl der Lebensdauer als auch der Selektivität erzielt wird, war nicht vorauszusehen. Während der verlängerten Aufheizphase sollten nämlich bei der niedrigeren Temperatur in kinetisch kontrollierter Reaktion zwar Veränderungen der Struktur des Katalysators eintreten können. Diese müßten jedoch im nachfolgenden, langdauernden Einsatz bei der höheren Betriebstemperatur in dieselbe, thermodynamisch günstigste Struktur übergehen, welche der Katalysator ohne die erfindungsgemäße Vorbehandlung annimmt.

Als Betriebstemperatur $T_R$ wird die Temperatur bezeichnet, bei der eine günstige und wirtschaftliche Umsetzung des Synthesegases zu den sauerstoffhaltigen Verbindungen Essigsäure, Acetaldehyd und Ethanol im Dauerbetrieb erfolgt. Sie kann in Abhängigkeit von der Katalysatorzusammensetzung, der Katalysatorherstellung, der Art und Menge der Promotoren, sowie in Abhängigkeit von den übrigen Reaktionsbedingungen wie Druck, Raumgeschwindigkeit und Zusammensetzung der Eingangsgase in weiten Grenzen schwanken. Im allgemeinen liegt $T_R$ zwischen 200° C und 450° C, vorzugsweise zwischen 250 und 375° C. Der Betriebsdruck liegt im allgemeinen zwischen 1 und 300 bar, vorzugsweise zwischen 20 und 200 bar.

Für die erfindungsgemäße Umsetzung von Synthesegas werden Katalysatoren verwendet, die auf einem Träger 0,1 bis 15 Gew.-%, vorzugsweise 0,5 bis 10 Gew.-% Rhodium als Metall oder in einer Wertigkeitsstufe unter drei, also als Komplexverbindung des nullwertigen Rhodiums oder als Salz oder Komplexverbindung des ein- oder zweiwertigen Rhodiums, enthalten. Gegebenenfalls können die Katalysatoren auch noch Promotoren oder Aktivatoren, insbesondere Magnesium in Kombination mit Halogenidionen, sowie Mangan enthalten. Außerdem können auch solche Stoffe anwesend sein, die die Selektivität zu den einzelnen sauerstoffhaltigen Produkten beeinflussen, also beispielsweise Eisen, Zirkon, Hafnium, Lanthan, Platin, Quecksilber, Molybdän, Wolfram, Uran oder Thorium.

Als Träger können handelsübliche Trägermaterialien mit unterschiedlichen spezifischen Oberflächen verwendet werden, vorzugsweise solche mit spezifischen Oberflächen zwischen 50 und 1000 $m^2/g$. Geeignet sind z. B. Kieselsäure, Silicate, Aluminiumoxid, Titandioxid, Zirkondioxid, Zeolithe, Thoriumoxid und Spinelle.

Zur Durchführung des erfindungsgemäßen Verfahrens wird der Katalysator in die Reaktionszone gebracht und zunächst bis auf eine Temperatur $T_O$, die 75 bis 125°C unterhalb der vorgesehenen Betriebstemperatur $T_R$ liegt, in beliebiger Geschwindigkeit aufgeheizt. Die weitere Temperaturerhöhung von $T_O$ auf $T_R$ wird in der Weise durchgeführt, daß man kontinuierlich oder in Temperaturintervallen von maximal 10°C aufheizt, wobei die Zeit für die Temperaturerhöhung von $T_O$ auf $T_R$ 100 bis zu 1000 Stunden, vorzugsweise 120 bis 800 Stunden beträgt. Wenn also beispielsweise die Betriebstemperatur bei 300°C liegt, wird nach Erreichen einer Katalysatortemperatur, die zwischen 175 und 225°C liegt, im weiteren Verlauf langsam weiter aufgeheizt, bis nach einer Zeitspanne von 100 bis 1000 Stunden die Betriebstemperatur von 300°C erreicht ist.

Während des Aufheizens, das unter normalem oder erhöhtem Druck erfolgt, leitet man Synthesegas oder ein Inertgas wie Stickstoff oder Gemische dieser Gase über den Katalysator. Vorteilhaft ist es jedoch, wenn noch vor Erreichen der niederen Temperaturstufe $T_O$ Synthesegas unter dem vorgesehenen Betriebsdruck über den Katalysator strömt, damit in dem für das erfindungsgemäße Verfahren wichtigen Temperaturbereich zwischen $T_O$ und $T_R$ ein zu schneller Temperaturanstieg bei Einsetzen der Reaktion vermieden wird.

Für die Verfahrensdurchführung können die herkömmlichen Festbettreaktoren verwenndet werden, wobei es zur besseren Wärmeabführung vorteilhaft ist, die Katalysatorschichtdicke gering zu halten. Geeignet sind auch Reaktoren mit bewegtem Katalysatorbett oder Wirbelbettreaktoren. Eine bevorzugte Ausführungsform besteht darin, die Umsetzung in einer Kreisgasapparatur durchzuführen, in der nach Abtrennung der kondensierbaren Reaktionsprodukte das nicht umgesetzte Gasgemisch nach Zugabe von frischem Synthesegas wieder in den Reaktor zurückgeführt wird.

Als Kreisgasapparaturen kommen solche mit innerem oder äußerem Gasumlauf in Betracht.

Die Erfindung soll durch die nachfolgenden Beispiele erläutert werden, wobei die Beispiele aber in keiner Weise einschränkend sein sollen. Die dort angegebenen Gasmengen beziehen sich auf Normalbedingungen.

Die angegebenen Raumzeitausbeuten (RZA, in g pro Liter Katalysator und Stunde) beziehen sich auf die Summe von Essigsäure AcOH, Acetaldehyd AcH und Ethanol EtOH.

## Vergleichsbeispiel 1

Zunächst wird ein Katalysator wie folgt hergestellt:

100 g Kieselsäure (Korngröße 1—3 mm) mit einem Schüttgewicht von 0,42 kg/l und einer BET-Oberfläche von 270 $m^2/g$ werden mit einer Lösung von

2,5 $MgCl_2 \cdot 6\,H_2O$

in 110 ml Wasser getränkt und 2 Stunden bei 80°C und 2 Stunden bei 150°C getrocknet und anschließend 30 Minuten bei 800°C gesintert. Nach dem Erkalten wird mit einer Lösung von

8 g $RhCl_3 \cdot x\,H_2O$ (37,8 Gew.-% Rh)

in 110 ml Wasser imprägniert und wie oben getrocknet. Der Katalysator wird durch 3stündiges Überleiten von 50 l/h Wasserstoff bei 250°C unter Normaldruck reduziert. Er enthält 2,8 Gew.-% Rh, 0,25% Mg und 1,4% Cl.

100 ml (45 g) dieses Katalysators werden in ein Reaktionsrohr von 1 m Länge und 16 mm innerem Durchmesser mit einem koaxial angebrachten Thermometerrohr von 6 mm äußerem Durchmesser gefüllt. Nach Spülen der Apparatur mit Stickstoff wird mit einem Kohlenmonoxid-Wasserstoff-Gemisch (Vol.-Verhältnis 1 : 1) ein Druck von 80 bar eingestellt und der Katalysator unter Überleiten von 200 l/h Synthesegas obiger Zusammensetzung aufgeheizt. Nach 2,5 Stunden ist eine Katalysatortemperatur von 200°C, nach insgesamt 4 Stunden eine solche von 250°C und nach insgesamt 7,5 Stunden die Betriebstemperatur von 300°C erreicht.

Es werden nun weiterhin stündlich 200 l Synthesegas (Kohlenmonoxid und Wasserstoff im Vol.-Verhältnis 1 : 1) bei 80 bar und 300°C über den Katalysator geleitet. Das Reaktionsgemisch wird in einem solegekühlten Kondensator auf +5°C gekühlt und das nicht kondensierte Restgas über ein Ventil entspannt. Kondensat und Abgas werden gaschromatographisch analysiert. Es werden auf umgesetztes CO, ermittelt:

| Laufzeit h | Temp. °C | RZA g/l · h | Selektivitäten (Mol. %) AcOH | AcH | EtOH | $CH_4$ |
|---|---|---|---|---|---|---|
| 4 | 250 | 170 | 76 | 5 | 1,5 | 9 |
| 7,5 | 300 | 380 | 70 | 7 | 2,3 | 13 |
| 100 | 300 | 375 | 68 | 7 | 3,5 | 16 |
| 1280 | 300 | 290 | 61 | 9 | 6,0 | 18 |

Restliches CO wird zu Kohlendioxid und höhermolekularen sauerstoffhaltigen Verbindungen umgesetzt.

Beispiel 1

100 ml frischer Katalysator der in Vergleichsbeispiel 1 beschriebenen Zusammensetzung werden in 2,5 Stunden auf 200° C und danach mit einer Temperatursteigerungsrate von 5° C in jeweils 10 Stunden innerhalb 200 Stunden bis auf 300° C aufgeheizt. Unter den ansonsten gleichen Bedingungen wie in Vergleichsbeispiel 1 werden nun folgende Raumzeitausbeuten und Selektivitäten erhalten.

| Laufzeit h | Temp. °C | RZA g/l · h | Selektivitäten (Mol. %) AcOH | AcH | EtOH | $CH_4$ |
|---|---|---|---|---|---|---|
| 102,5 | 250 | 180 | 64 | 10 | 5 | 12 |
| 200 | 300 | 385 | 75 | 6 | 3 | 9 |
| 1280 | 300 | 375 | 76 | 5 | 3 | 10 |

Beispiel 2

Man verfährt wie in Beispiel 1, erhöht jedoch die Katalysatortemperatur ab 200° C mit einer Temperatur-Steigerungsrate von 2° C in jeweils 10 Stunden im Verlauf von 500 Stunden bis auf 300° C. Zu diesem Zeitpunkt beträgt die Raumzeitausbeute 370 g/lh und nach insgesamt 1280 Stunden noch 355 g/lh bei unveränderter Essigsäure-Selektivität.

Vergleichsbeispiel 2

Der Katalysator wird wie folgt hergestellt: 100 g Kieselsäure mit 270 $m^2/g$ BET-Oberfläche und 1,22 ml/g Porenvolumen werden mit einer Lösung von

11,1 g $RhCl_3 \cdot x\ H_2O$ (37,8 Gew.-% Rh)

in 115 ml Waser getränkt, 4 Stunden bei 120° C getrocknet und anschließend durch 3stündiges Überleiten von 30 l/h Wasserstoff bei 450° C unter Normaldruck reduziert. Er enthält 4,0 Gew.-% Rhodium und 0,6 Gew.-% Chlor.

Die Apparatur ist die gleiche wie in Vergleichsbeispiel 1. In das Reaktionsrohr werden 100 ml des oben beschriebenen Katalysators gefüllt. Nach dem Spülen der Apparatur mit Stickstoff leitet man 200 l/h eines Kohlenmonoxid-Wasserstoff-Gemisches (Vol.-Verhältnis 1 : 1) bei 100 bar über den Katalysator und heizt innerhalb 24 Stunden bis auf 340° C auf. Danach werden weiterhin stündlich 200 l des Gasgemisches bei 100 bar und 340° C über den Katalysator geleitet. Man erhält folgende Raumzeitausbeuten bzw. Selektivitäten:

| Laufzeit h | Temp. °C | RZA g/lh | Selektivität (Mol. %) AcOH | AcH | EtOH | $CH_4$ |
|---|---|---|---|---|---|---|
| 10 | 250 | 41 | 35 | 12 | 24 | 23 |
| 24 | 340 | 156 | 31 | 8 | 21 | 33 |
| 100 | 340 | 122 | 27 | 8 | 17 | 38 |
| 570 | 340 | 72 | 23 | 6 | 10 | 46 |

Beispiel 3

Es wird wie im Vergleichsbeispiel 2 verfahren. Nach 10 Stunden (vom Beginn des Aufheizens an gerechnet) ist eine Katalysatortemperatur von 250°C erreicht. Danach wird die Temperatur nur noch um 6°C in je 10 Stunden erhöht, bis man nach insgesamt 160 Stunden die gleiche Betriebstemperatur von 340°C erreicht wie in Vergleichsbeispiel 2. Folgende Raumzeitausbeuten und Selektivitäten werden erzielt:

| Laufzeit h | Temp. °C | RZA g/lh | Selektivität (Mol. %) AcOH | AcH | EtOH | $CH_4$ |
|---|---|---|---|---|---|---|
| 10 | 250 | 39 | 33 | 14 | 22 | 22 |
| 100 | 340 | 168 | 39 | 11 | 21 | 19 |
| 960 | 340 | 152 | 42 | 10 | 23 | 18 |

**Patentansprüche**

1. Verfahren zur Herstellung von Essigsäure, Acetaldehyd und Ethanol durch Umsetzung von Kohlenmonoxid und Wasserstoff in der Gasphhase an Rhodium und gegebenenfalls Promotoren enthaltenden Katalysatoren bei erhöhten Temperaturen und erhöhten Drücken, dadurch gekennzeichnet, daß man in der Anfangsphase des Verfahrens während des Aufheizens des Katalysators auf die für den Dauerbetrieb vorgesehene Betriebstemperatur $T_R$ die Temperaturerhöhung im Temperaturbereich zwischen $T_O$ und $T_R$, wobei $T_O$ 75 bis 125°C unterhalb $T_R$ liegt, kontinuierlich oder in Temperaturstufen von maximal 10°C und in einem Zeitraum von 100 bis 1000 Stunden vornimmt, wobei die Temperatur in einem beliebigen 10-Stunden-Intervall um maximal 10°C erhöht wird.

2. Verfahren nach Anspruch 1, dadurch gekennzeichnet, daß man die Temperaturerhöhung in einem Zeitraum von 120 bis 800 Stunden vornimmt.

**Claims**

1. A process for the manufacture of acetic acid, acetic aldehyde and ethanol by reaction in the gaseous phase of carbon monoxide and hydrogen in the presence of catalysts containing rhodium and optionally promoters, at elevated temperature and pressure, which is characterized by raising the temperature in the starting phase of the process, where the catalyst is heated to the working temperature $T_R$ intended for continuous operation, in a range of from $T_O$ to $T_R$, $T_O$ being 75 to 125°C below $T_R$, continuously or in steps of 10°C at most, in a period of time of from 100 to 1,000 hours; the temperature being raised by 10°C at most within any 10 hours interval.

2. The process as claimed in Claim 1, which is characterized by raising the temperature in a period of time of 120 to 800 hours.

**Revendications**

1. Procédé de préparation d'acide acétique, d'acétaldéhyde et d'éthanol par réaction de monoxyde de carbone et d'hydrogène, en phase gazeuse, en présence de catalyseurs contenant du rhodium et éventuellement des promoteurs, à des températures élevées et sous des pressions élevées, procédé

caractérisé en ce que, dans la phase initiale du procédé, au cours du chauffage du catalyseur jusqu'à la température $R_R$ prévue pour le fonctionnement en régime, on fait monter la température, dans l'intervalle allant de $T_O$ à $T_R$, la température $T_O$ étant inférieure de 75 à 125° C à la température $T_R$, continuellement ou par étapes d'au plus 10° C et pendant une durée de 100 à 1000 heures, la température étant élevée d'au plus 10° C dans un intervalle quelconque de 10 heures.

2. Procédé selon la revendication 1, caractérisé en ce que l'augmentation de température est effectuée en un temps de 120 à 800 heures.